(19) Europäisches Patentamt
European Patent Office
Office européen des brevets

(11) Publication number: **0 059 108**
**B1**

(12) # EUROPEAN PATENT SPECIFICATION

(45) Date of publication of patent specification: **14.08.85**

(51) Int. Cl.[4]: **C 07 C 103/84**, A 61 K 31/165

(21) Application number: **82300929.5**

(22) Date of filing: **23.02.82**

(54) **Derivatives of dihydroxybenzoic acid.**

(30) Priority: **24.02.81 JP 25991/81**

(43) Date of publication of application:
**01.09.82 Bulletin 82/35**

(45) Publication of the grant of the patent:
**14.08.85 Bulletin 85/33**

(84) Designated Contracting States:
**BE CH DE FR GB IT LI NL**

(56) References cited:
**JP-A-43 004 338**

**Chemical Abstracts, vol. 85, no. 23, 6 December 1976, Columbus, Ohio, USA, V. ARIESAN et al. "Investigations on some synthetic antitrichomoniasis drugs (2,5-dihydrobenzoic acid N-substituted amides)",page 486, column 2, abstract no. 177027v**
**Chemical Abstracts, vol. 85, no. 19, 8 November 1976, Columbus, Ohio, USA, H. NISHIMURA et al. "Benzanilide derivatives", page 502, column 1, abstract no. 142860j**

(73) Proprietor: **KUREHA KAGAKU KOGYO KABUSHIKI KAISHA**
**9-11 Horidome-cho 1-chome Nihonbashi Chuo-ku**
**Tokyo (JP)**

(72) Inventor: **Takita, Hitoshi**
**1-8 Toyotama-kita**
**Nerima-ku Tokyo (JP)**
Inventor: **Mukaida, Yutaka**
**No. 21 Daiichi-Kurehasoh 3-10-13 Nerima**
**Nerima-ku Tokyo (JP)**
Inventor: **Noda, Sakuo**
**No. 103 Kureha Ohkubo-Shataku 3-26-1**
**Hyakunin-cho Shinjuku-ku Tokyo (JP)**
Inventor: **Kobayashi, Hidetoshi**
**2-21-2 Zenpukuji**
**Suginami-ku Tokyo (JP)**

(74) Representative: **Myerscough, Philip Boyd et al**
**J.A.Kemp & Co. 14, South Square Gray's Inn**
**London, WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 059 108 B1

**Description**

The present invention relates to derivatives of dihydroxybenzoic acid, their preparation and pharmaceutical compositions containing them.

Japanese Patent Publication No. 43—4338(1968) discloses trans-4-(N-salicyloyl-aminomethyl)cyclohexane-1-carboxylic acid.

Chemical Abstracts volume 85, 1976, 142860j discloses benzanilides of formula:

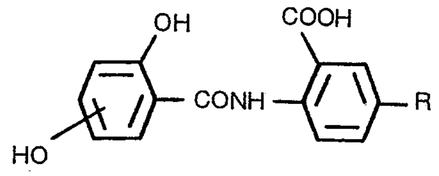

in which R represents H or OH. These benzanilides are said to have antitubercular, analgesic, antiinflammatory and uric-acid excreting activities (no data).

According to the present invention there are provided derivatives of dihydroxybenzoic acid (hereinafter referred to as the present compounds) represented by the formula (I):

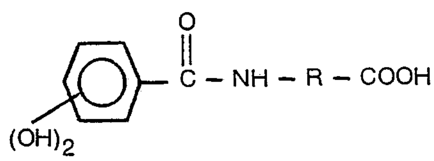

(I)

wherein R represents

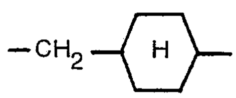 or $-(CH_2)_5-$ ;

and salts and esters thereof. In the general formula (I), the forms of the cyclohexane ring include both trans- and cis- forms.

4-[N-(2',3'-dihydroxybenzoyl)aminomethyl]cyclohexane-1-carboxylic acid,
4-[N-(2',4'-dihydroxybenzoyl)aminomethyl]cyclohexane-1-carboxylic acid,
4-[N-(2',5'-dihydroxybenzoyl)aminomethyl]cyclohexane-1-carboxylic acid,
4-[N-(2',6'-dihydroxybenzoyl)aminomethyl]cyclohexane-1-carboxylic acid,
4-[N-(3',4'-dihydroxybenzoyl)aminomethyl]cyclohexane-1-carboxylic acid,
4-[N-(3',5'-dihydroxybenzoyl)aminomethyl]cyclohexane-1-carboxylic acid,
6-[N-(2',3'-dihydroxybenzoyl)amino]caproic acid,
6-[N-(2',4'-dihydroxybenzoyl)amino]caproic acid,
6-[N-(2',5'-dihydroxybenzoyl)amino]caproic acid,
6-[N-(2',6'-dihydroxybenzoyl)amino]caproic acid,
6-[N-(3',4'-dihydroxybenzoyl)amino]caproic acid,
6-[N-(3',5'-dihydroxybenzoyl)amino]caproic acid.

Compounds of the invention include salts and esters of the derivatives of dihydroxybenzoic acid of formula (I). The salts are preferably alkali or alkaline earth metal salts such as the sodium, potassium calcium or magnesium salt, or substituted- or unsubstituted ammonium salts. The esters are preferably alkyl esters in which the alkyl group has 1 to 3 carbon atoms, such as methyl, ethyl, or n- or iso-propyl.

The present compounds and their salts and esters have excellent pharmacological activity such as inhibitory effects on platelet aggregation and polynuclear leukocyte migration and are pharmacologically safe. Accordingly, they are important compounds for use as pharmaceuticals in treating various diseases such as inflammation and thrombosis and are industrially important as intermediates for medicaments.

According to the present invention, derivatives of formula (I) and salts and esters thereof are preferably prepared according to the following reaction scheme:

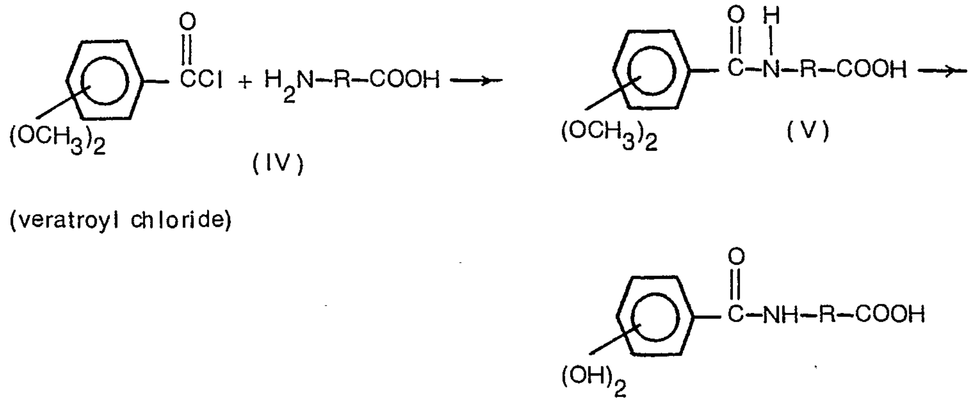

(IV)

(veratroyl chloride)

(V)

(I)

wherein R is as defined above, and optionally converting the derivative of dihydroxybenzoic acid of formula (I) to a salt or ester thereof. The first reaction, called the Schotten-Bauman reaction, is suitably carried out in a solvent such as a mixed solvent of a polar solvent (e.g. dioxane) and water in the presence of 2 to 3 equivalents of sodium hydroxide, based on veratroyl chloride at a temperature of 5 to 30°C. The second reaction is suitably carried out in the presence of a de-methylation agent. The reaction temperature is preferably 80 to 130°C when anhydrous aluminum trihalide such as $AlCl_3$ or $AlBr_3$ is used as the de-methylation agent in a molar amount of 5 to 10, and is preferably −78 to 12°C when anhydrous boron trihalide is used in a molar amount of more than 2.

The present invention provides a further process for the preparation of derivatives of formula (I) and salts and esters thereof, which process comprises:

(i) reacting a dihydroxybenzoic acid chloride represented by the formula (II):

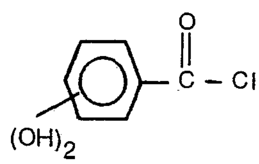

(II)

with a hydrochloric acid salt of an aminocarboxylic acid derivative represented by the formula (III):

$$NH_2—R—COOH.HCl \qquad (III)$$

wherein R is as defined above, or an ester, preferably a $C_1$—$C_3$ alkyl ester, thereof; and

(ii) when the resulting compound is a derivative of formula (I), optionally neutralizing said derivative with a base to form a salt thereof.

Step (i) is suitably carried out in the presence of a de-hydrochlorination agent such as a trialkylamine, in which the alkyl groups have 1 to 4 carbon atoms, in a molar amount of more than 2 at a temperature of 5 to 30°C.

The salt form of the present compound is prepared in step (ii) by the conventional method for neutralization by using a base such as a hydroxide, carbonate or bicarbonate of an alkali or an alkaline earth metal, for example sodium, potassium, calcium or magnesium, or ammonium, or a primary-, secondary- or tertiary amine.

The present compounds and their salts and esters show an inhibitory effect on platelet aggregation and/or polynuclear leukocyte migration and a low acute toxicity, as is shown in the Examples below. Accordingly, the present compound is useful by itself or as an active ingredient in a pharmaceutical composition for treating several diseases such as inflammation, thrombosis, cerebral hemorrhage, hypertension and asthma, and further as an intermediate for medicaments.

The present invention therefore also provides a pharmaceutical composition which comprises a derivative of a dihydroxybenzoic acid represented by the formula (I) or a pharmaceutically acceptable salt or ester thereof as active ingredient, together with a pharmaceutically acceptable carrier. The pharmaceutical composition may be in unit dosage form comprising a therapeutically effective amount of a derivative of formula (I) or a pharmaceutically acceptable salt or ester thereof.

When the compounds of the invention are used in a pharmaceutical composition in the form of the salt or ester, these must be pharmaceutically acceptable.

The compounds of the invention can be administered perorally, rectally or by injection in the various dosage forms as a composition together with a pharmaceutically acceptable carrier and/or an adjuvant. In these cases, a mixture of two or more compounds of the invention or a mixture together with other pharmaceutically active materials may be used as active ingredient in a pharmaceutical composition.

The dosage form of the composition may be as a tablet, sublingual tablet, powder, capsule, troch, aqueous or oily solution, suspension, emulsion, syrup, aqueous or oily injection. Examples of the carrier

mentioned above include water, gelatin, lactose, starch, pectin, magnesium stearate, stearic acid, talc, vegetable oil, gum arabic, polyalkylene glycol, vaseline, sorbitan trioleate, polyoxyethylene-sorbitan monooleate, alkylphenol, aliphatic alcohol, polyvinylpyrrolidone or the like. In the composition, if necessary, an edulcorant, flavor, tinctorial agent, preservative, salt for osmoregulation or buffer, that is a conventional pharmaceutical adjuvant, may be used.

The content of a compound(s) of the invention in a pharmaceutical composition may be varied as desired, for example from 0.01% to 100% by weight, preferably from 0.05% to 80% by weight of the composition.

The pharmaceutical compositions can be administered to a human or animal parenterally, for example, rectally, by injection (hypodermic, intramuscular or intravenous, or drip), preferably perorally (for example sublingually).

A dose of the pharmaceutical composition of the present invention is suitably 0.1 to 500 mg, preferably 0.5 to 200 mg, per day per kilogram of body weight in the case of peroral administration of a human, and 0.01 to 200 mg, preferably 0.1—100 mg, per day per kilogram of body weight in the case of parenteral administration. A composition can be administered from one to four times a day. However the dose will depend on age, individuality, the condition of a disease, etc. of a human or animal, and doses outside the ranges may be used.

The following Examples illustrate the invention. Percentages are by weight unless otherwise indicated.

Example 1

Preparation of trans-4-[N-(3′,4′-dihydroxybenzoyl)aminomethyl]cyclohexane-1-carboxylic acid

10 Grams (58.1 mmol) of protocatechuic acid and 20 ml of dehydrated and purified ethylether were introduced into a 100 ml flask. While stirring, 25 g (210 mmol) of thionyl chloride was added dropwise into the flask under an atmosphere of nitrogen. After stirring the mixture for 24 hours at room temperature to cause reaction, ether and excess thionyl chloride were evaporated off to obtain a viscous liquid, pale yellow in colour. After adding 50 ml of dehydrated ether to the mixture and then removing the thus separated colourless powder by filtration, the solvent was evaporated off from the filtrate to obtain 7.5 g of crude protocatechuic acid chloride as a viscous pale yellow liquid.

Next, 7.5 g of the thus obtained protocatechuic acid chloride, 12.6 g (65.4 mmol) of ethyl trans-4-aminomethylcyclohexane-1-carboxylate.HCl, 16 g of triethylamine and 60 ml of benzene were introduced into a 200 ml three-necked flask. After stirring the mixture for 2 hours at room temperature under an atmosphere of nitrogen to cause reaction, the solvent was evaporated off to obtain 30 g of residue. After adding 45 ml of distilled water to the residue and then making the residue acidic with the addition of 1N hydrochloric acid, the residue was extracted with ethyl acetate.

By dehydrating the residue and removing the solvent from the extract, 10.35 g of crude product of the desired compound was obtained, which was hydrolyzed with 21 ml of 4N sodium hydroxide solution and 60 ml of ethanol by heating under reflux. After removing the solvent from the hydrolyzed product, it was acidified with 1N hydrochloric acid and extracted with ethyl acetate. By dehydrating and removing the solvent from the extract, 8 g of crude trans-4-[N-(3′,4′-dihydroxybenzoyl)aminomethyl]cyclohexane-1-carboxylic acid was obtained. By purifying the crude product through silica gel-column chromatography and recrystallization, 3.1 g of purified product was obtained in the form of a colourless powder.

The characteristics of the desired compound (acid form) thus obtained were as follows:

(1) melting point on a hot plate: 212 to 214°C

(2) elementary analysis:

| | C(%) | H(%) | N(%) |
|---|---|---|---|
| theoretical: | 61.42 | 6.60 | 4.80 |
| experimental: | 61.2 | 6.53 | 4.77 |

(3) nuclear magnetic resonance (NMR) spectrum in dimethyl sulfoxide;

δ = 0.86—1.84 (9H, m), 1.99—2.08 (1H), 3.06 (2H), 6.75 (1H, d), 7.21 (1H, d), 7.28 (1H, s), 8.10 (1H), 8.56—10.56 (3H).

(4) infrared absorption (IR) spectrum by KBr tablet method: as shown in Figure 1 of the accompanying drawings.

Example 2

Preparation of 6-[N-(3′,4′-dihydroxybenzoyl)amino]caproic acid

10.0 Grams (54.9 mmol) of veratric acid was dissolved in 50 ml of benzene in a 2-neck 100 ml-round bottom flask. After adding 2 to 3 drops of pyridine to the solution, 13 g (109 mmol) of thionyl chloride was added dropwise into the solution while stirring the solution at room temperature within 3 min. Then the contents of the flask were heated to 70 to 80°C on a water bath, and maintained at that temperature for 2 hours under a reflux condenser and agitation. Then, the solvent and excess thionyl chloride were

evaporated off under a reduced pressure to obtain 11.0 g of veratroyl chloride as colourless powder in a yield of 100%.

9.0 Grams (68.8 mmol) of 6-aminocaproic acid was dissolved in 55 ml of distilled water in a 200 ml-beaker, and 17 ml of dioxan and 17 ml of 4N sodium hydroxide solution were added to the solution.

While immersing the beaker in a water bath and stirring the solution in the beaker, 11.0 g of veratroyl chloride (obtained above) and 17 ml of 4N sodium hydroxide solution were added to the solution gradually while maintaining the temperature of the mixture not higher than 30°C to obtain a transparent reaction mixture of pale yellow colour. By pouring the reaction mixture into 9 ml of concentrated hydrochloric acid in a 200-ml beaker, colourless powdery crystals separated out in the beaker. After collecting the crystals and recrystallizing from 275 ml of a mixed solvent of water and ethanol (mixing ratio; 10:2), 12.7 g of 6-[N-(3',4'-dimethoxybenzoyl)amino]caproic acid was obtained as colourless acicular crystals melting at 137.0 to 138.0°C in a yield of 78.4%. The elementary analytical data on the thus prepared compound were as follows:

| | C(%) | H(%) | N(%) |
|---|---|---|---|
| experimental: | 60.70 | 7.20 | 4.70 |
| theoretical: | 61.00 | 7.17 | 4.74 |

Next, 6.0 g (20.3 mmol) of the thus obtained 6-[N-(3',4'-dimethoxybenzoyl)amino]caproic acid and 100 g of boron trichloride were introduced into a 200 ml-pearshaped flask. The mixture was left to react at −78°C in a dry-ice/methanol bath for 3 days under moistureless conditions. After bringing the reaction mixture to room temperature and evaporating off the boron trichloride under a normal pressure, 30 ml of distilled water was added to the reaction mixture and the mixture was stirred at room temperature. After collecting the thus separated colourless powdery insoluble material by filtering the mixture and drying the powdery material, 40 ml of distilled water was added to the material. The mixture was stirred for one hour at room temperature to dissolve the material in water. After removing the remaining water-insoluble fractions by filtration, the filtrate was freeze-dried to obtain 1.716 g of 6-[N-(3',4'-dihydroxybenzoyl)amino]-caproic acid as a colourless powder in a yield of 31.6%.

The characteristics of the compound thus obtained were as follows:

(1) melting point on a capillary; 113.0—115.0°C

(2) elementary analysis;

| | C(%) | H(%) | N(%) |
|---|---|---|---|
| theoretical: | 58.42 | 6.41 | 5.24 |
| experimental: | 58.30 | 6.55 | 5.40 |

(3) infrared absorption (IR) spectrum by KBr tablet method: as shown in Figure 2 of the accompanying drawings.

## Example 3

Pharmacological activities

*Inhibitory effect on platelet aggregation:*

Rabbit platelet rich plasma (PRP) was used for Aggregation Tests. PRP was prepared by centrifugation of blood collected from the ear vein of a rabbit and diluted to a number-concentration 300,000/µl with platelet poor plasma. Platelet aggregation was induced by sodium arachidonate and monitored (measured) with a four channel platelet aggregation Tracer PAT—4A (Niko Bioscience Co., Japan). Aggregation Tracer tubes each containing 230 µl PRP were preincubated at 37°C for 5 min with each specimen.

The results are shown in Table 1.

*Inhibitory effect on polynuclear leukocyte migration:*

Inhibitory effect on polynuclear leukocyte migration was examined by using male rats (Donryu) of body weight of about 150 g and Granuloma CMC (carboxymethylcellulose) pouch method (refer to Yakugaku Zasshi, *88*, 1472, (1968)).

After shaving the hair from the dorsum of each rat over an area about 5 cm in diameter, 5 ml of air was hypodermically injected into each site thus prepared to form a pouch. After twenty four hours, 5 ml of aqueous 2% (w/v) solution CMC sodium salt at 37°C was injected into each pouch. At the same time an aqueous solution of a respective specimen was administered perorally to the rat.

After the injection of CMC, the liquid in the pouch was collected as time passed by in an amount of 0.5 ml, and stained. Then the number of polynuclear leukocytes which had migrated into each pouch was counted.

The results are shown in Table 1.

*Prophylactic effect on adjuvant arthritis:*

The prophylactic effect on adjuvant arthritis was examined using respective groups each of 6 female JCL—SD rats 8 weeks old, and the conventional method (refer to Fujihira *et al.*, Pharmacometrics, *5*(2), 169—182, (1971)). Freund's complete adjuvant (0.6 mg/0.1 ml) was inoculated into the right hind paw of etherized rats. After palinesthesia, each specimen was administered perorally once a day for 14 continuous days.

The results are shown in Table 1.

TABLE 1

| | Specimen | |
|---|---|---|
| | Trans-4-[N-(3',4'-dihydroxy-benzoyl)aminomethyl]cyclo-hexane-1-carboxylic acid | Sodium trans-4-(N-salicy-loyl-aminomethyl)cyclo-hexane-1-carboxylic acid — the comparative compound of JP—A—43-4338 (1968) |
| Inhibition rate of platelet aggregation (%) 1) | 100 | 10 |
| Inhibition rate of polynuclear leukocyte migration (%) 2) | 35 | 5 |
| Prophylactic effect on adju-vant arthritis (%) 3) | 30 | 5 |

Notes: 1) The average value of inhibition rate (%) of platelet aggregation at the concentration of 1.5 mM of each Specimen.

2) The average value of inhibition rate (%) of polynuclear leukocyte migration at the dose of 100 mg p.o./kg body weight of each Specimen determined at 6 hours after the administration.

3) The average value of prophylactic rate (%) on the adjuvant arthritis at the dose of 100 mg p.o./kg/day for 14 continuous days.

*Acute toxicity:*

Acute toxicity was examined by administering perorally an aqueous solution of each specimen to female JCL—ICR mice 5 to 6 weeks old. The specimens were trans-4-[N-(3′,4′-dihydroxybenzoyl)aminomethyl]cyclohexane-1-carboxylic acid, and 6-[N-(3′,4′-dihydroxybenzoyl)amino]caproic acid. Median lethal dose ($LD_{50}$) of each specimen was more than 3000 mg/kg, and no abnormalities were found in the mice.

As shown above, the compounds of the invention have inhibitory effects on platelet aggregation and polynuclear leukocyte migration, and a low toxicity. Accordingly, the compounds are useful for treating various diseases such as inflammation, thrombosis, asthma or cancer, especially chronic diseases such as rheumatism or systemic lupus erythematosus (SLE).

Example 4

Manufacture of a pharmaceutical composition in the form of capsules

Ten parts by weight of trans-4-[N-(3′,4′-dihydroxybenzoyl)aminomethyl]cyclohexane-1-carboxylic acid, 15 parts by weight of heavy magnesium oxide and 75 parts by weight of lactose were uniformly mixed to form a powder, and by putting the thus prepared powder into gelatin capsules, capsulates were prepared.

Example 5

Manufacture of a pharmaceutical composition in the form of granules

After mixing uniformly and then kneading 45 parts by weight of trans-4-[N-(3′,4′-dihydroxybenzoyl)-aminomethyl]cyclohexane-1-carboxylic acid, 10 parts by weight of starch, 20 parts by weight of lactose, 3 parts by weight of polyvinyl alcohol and 22 parts by weight of water, the mixture thus formed was crushed and formulated into granules which were then dried and sifted.

Example 6

Manufacture of an injectable pharmaceutical composition

0.6 Parts by weight of sodium trans-4-[N-(3′,4′-dihydroxybenzoyl)aminomethyl]cyclohexane-1-carboxylate was added to 99.4 parts by weight of aqueous physiological saline solution. After heating the mixture to form a clear solution was sterilized.

**Claims**

1. A derivative of dihydroxybenzoic acid represented by the formula (I):

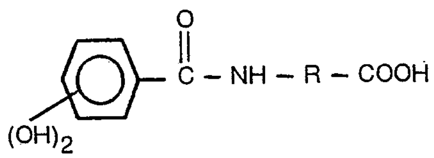

(I)

wherein R represents

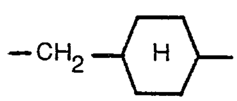 or $-(CH_2)_5-$ ;

or a salt or an ester thereof.

2. A compound according to claim 1, which is trans 4-[N-(3′,4′-dihydroxybenzoyl)aminomethyl]cyclohexane-1-carboxylic acid.

3. A compound according to claim 1, which is 6-[N-(3′,4′-dihydroxybenzoyl)amino]caproic acid.

4. A process for the preparation of a derivative of dihydroxybenzoic acid represented by the formula (I) as defined in claim 1 or a salt or an ester thereof, which process comprises

(i) reacting veratroyl chloride with a compound of formula (IV):

$$H_2N—R—COOH \quad (IV)$$

wherein R is as defined in claim 1;

(ii) converting the resulting compound of formula (V)

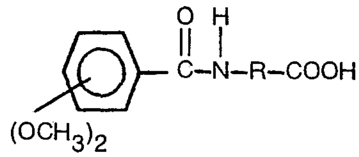

(V)

wherein R is as defined above, to a derivative of dihydroxybenzoic acid of formula (I); and

(iii) optionally converting the derivative of dihydroxybenzoic acid of formula (I) to a salt or ester thereof.

5. A process for the preparation of a derivative of a dihydroxybenzoic acid represented by the formula (I) as defined in claim 1 or a salt or an ester thereof, which process comprises (i) reacting a dihydroxybenzoic acid chloride represented by the formula (II):

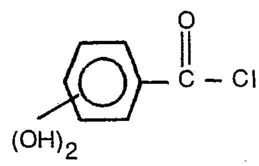

(II)

with a hydrochloric acid salt of an aminocarboxylic acid derivative represented by the formula (III):

$$NH_2—R—COOH.HCl \qquad (III)$$

wherein R is as defined in claim 1, or an ester thereof; and (ii) when the resulting compound is a derivative of formula (I), optionally neutralizing said derivative with a base to form a salt thereof.

6. A pharmaceutical composition which comprises a derivative of a dihydroxybenzoic acid represented by the formula (I) as defined in claim 1 or a pharmaceutically acceptable salt or ester thereof as active ingredient, together with a pharmaceutically acceptable carrier.

7. A pharmaceutical composition in unit dosage form comprising a therapeutically effective amount of a derivative of dihydroxybenzoic acid represented by the formula (I) as defined in claim 1 or a pharmaceutically acceptable salt or ester thereof, and a pharmaceutically acceptable carrier.

**Patentansprüche**

1. Derivat der Dihydroxybenzoesäure entsprechend der Formel (I):

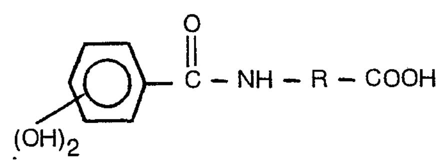

(I)

in der

R $-CH_2-$ 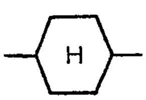 oder $-(CH_2)_5-$

bedeutet, oder ein Salz oder ein Ester desselben.

2. Verbindung nach Anspruch 1, dadurch gekennzeichnet, daß sie trans-4-[N-(3',4'-Dihydroxybenzoyl)-aminomethyl]cyclohexan-1-carbonsäure ist.

3. Verbindung nach Anspruch 1, dadurch gekennzeichnet, daß sie 6-[N-(3',4'-Dihydroxybenzoyl)-amino]capronsäure ist.

4. Verfahren zur Herstellung eines Derivats der Dihydroxybenzoesäue gemäß Formel (I) in Anspruch 1 oder eines Salzes oder eines Esters desselben, dadurch gekennzeichnet, daß

(i) Veratroylchlorid mit einer Verbindung der Formel (IV)

$$H_2N—R—COOH \qquad (IV),$$

in der R die in Anspruch 1 angegebene Bedeutung hat, zur Reaktion gebracht wird,

(ii) die resultierende Verbindung der Formel (V)

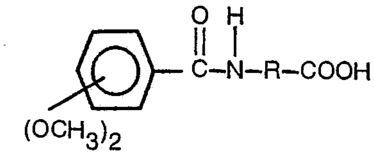

(V)

in der R die oben angegebene Bedeutung hat, in ein Derivat der Dihydroxybenzoesäure der Formel (I) umgewandelt wird und

(iii) gegebenenfalls das Derivat der Dihydroxybenzoesäure der Formel (I) in ein Salz oder einen Ester desselben umgewandelt wird.

5. Verfahren zur Herstellung eines Derivats einer Dihydroxybenzoesäure gemäß der Formel (I) in Anspruch 1 oder eines Salzes oder eines Esters desselben, dadurch gekennzeichnet, daß

(i) ein Dihydroxybenzoesäurechlorid gemäß der Formel (II)

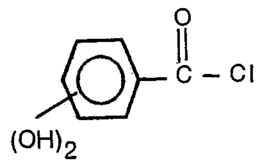

(II)

mit einem Salzsäuresalz eines Aminocarbonsäurederivats gemäß der Formel (III)

$NH_2—R—COOH.HCl$ (III),

in der R die in Anspruch 1 angegebene Bedeutung besitzt, oder einem Ester desselben zur Reaktion gebracht wird und

(ii) das Derivat gegebenenfalls mit einer Base neutralisiert wird, um ein Salz desselben zu bilden, wenn die resultierende Verbindung ein Derivat der Formel (I) ist.

6. Pharmazeutische Zusammensetzung, die ein Derivat einer Dihydroxybenzoesäure gemäß der Formel (I) in Anspruch 1 oder ein pharmazeutisch akzeptables Salz oder einen pharmazeutisch akzeptablen Ester desselben als aktiven Wirkstoff zusammen mit einem pharmazeutisch akzeptablen Täger enthält.

7. Pharmazeutische Zusammensetzung in Form von Dosierungseinheiten, die eine therapeutisch wirksame Menge eines Derivats der Dihydroxybenzoesäure gemäß der Formel (I) in Anspruch 1 oder ein pharmazeutisch akzeptables Salz oder einen pharmazeutisch akzeptablen Ester desselben und einen pharmazeutisch akzeptablen Träger enthält.

**Revendications**

1. Dérivé d'acide dihydroxybenzoïque représenté par la formule (I):

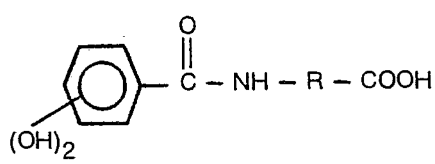

(I)

dans laquelle: R représente

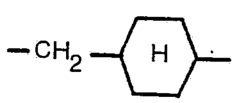 ou $-(CH_2)_5-$ ;

ou un ester ou sel de ce dérivé.

2. Composé selon la revendication 1, caractérisé par le fait qu'il est l'acide trans-4-[N-(3',4'-dihydroxybenzoyl)aminométhyl]cyclohexane-1-carboxylique.

3. Composé selon la revendication 1, caractérisé par le fait qu'il est l'acide 6-[N-(3',4'-dihydroxybenzoyl)amino]caproïque.

4. Procédé pour la préparation d'un dérivé d'acide dihydroxybenzoïque représenté par la formule (I) comme défini à la revendication 1, ou un sel ou un ester de ce dérivé, procédé consistant:
(i) à faire réagir du chlorure de vératroyle avec un composé de formule (IV):

$$H_2N—R—COOH \qquad (IV)$$

où R est défini comme dans la revendication 1;
(ii) à convertir le composé résultant de formule (V)

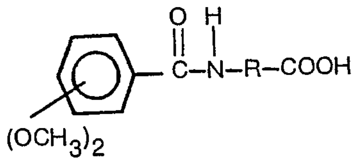

(V)

où R est défini comme ci-dessus, en un dérivé d'acide dihydroxybenzoïque de formule (I), et
(iii) à transformer éventuellement le dérivé d'acide dihydroxybenzoïque de formule (I) en un sel ou ester de ce dérivé.

5. Procédé pour la préparation d'un dérivé d'acide dihydroxybenzoïque représenté par la formule (I), tel que défini dans la revendication 1, ou un sel ou un ester de ce dérivé, procédé consistant (i) à faire réagir un chlorure d'acide dihydroxybenzoïque représenté par la formule (II):

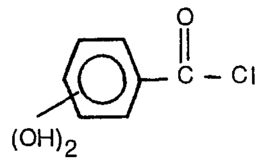

(II)

avec un chlorhydrate d'un dérivé d'acide aminocarboxylique représenté par la formule (III):

$$NH_2—R—COOH.HCl \qquad (III)$$

dans laquelle R est défini comme dans la revendication 1, ou un ester de ce composé: et (ii) lorsque le composé résultant est un dérivé de formule (I), à neutraliser éventuellement ledit dérivé par une base pour former un sel.

6. Composition pharmaceutique comprenant un dérivé d'acide dihydroxybenzoïque représenté par la formule (I) comme défini dans la revendication 1, ou un sel ou un ester pharmaceutiquement acceptables de ce dérivé, comme ingrédient actif, en mélange avec un support pharmaceutiquement acceptable.

7. Composition pharmaceutique sous forme de dosage unitaire comprenant une proportion efficace du point de vue thérapeutique d'un dérivé d'acide dihydroxybenzoïque représenté par la formule (I), tel que défini dans la revendication 1, ou un sel ou un ester pharmaceutiquement acceptable de ce dérivé, et un support pharmaceutiquement acceptable.

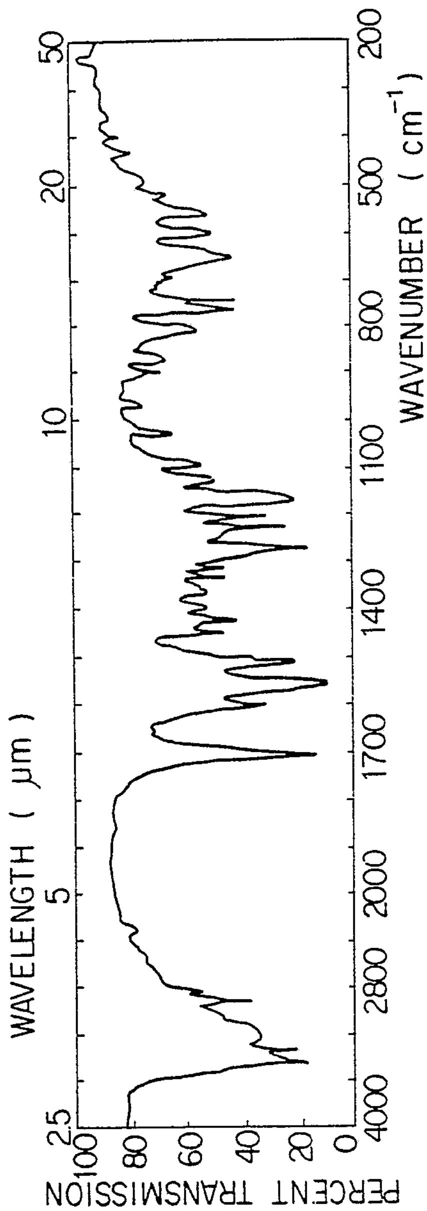
WAVELENGTH ( µm )
2.5
5
10
20
50
PERCENT TRANSMISSION
100
80
60
40
20
0
4000
2800
2000
1700
1400
1100
800
500
200
WAVENUMBER ( cm⁻¹ )

FIG. 1

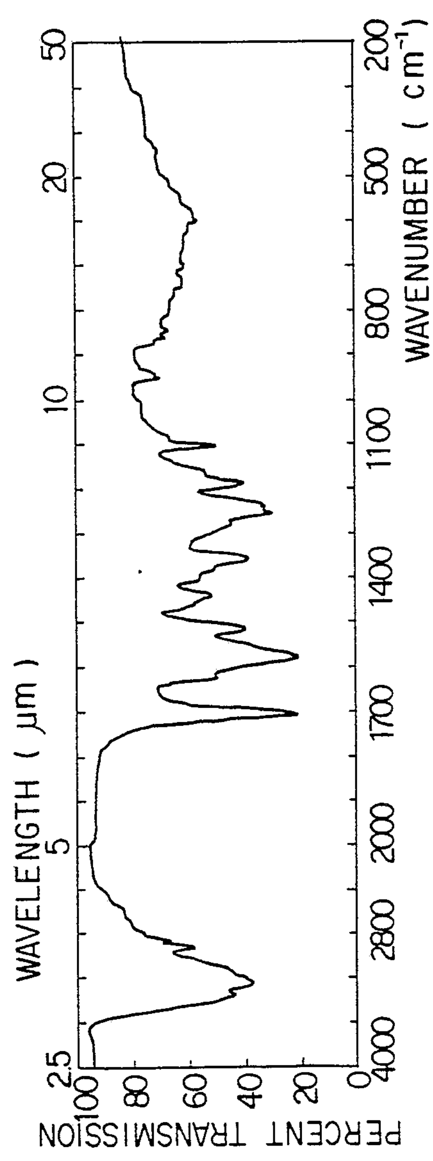
WAVELENGTH ( µm )
2.5
5
10
20
50
PERCENT TRANSMISSION
100
80
60
40
20
0
4000
2800
2000
1700
1400
1100
800
500
200
WAVENUMBER ( cm⁻¹ )

FIG. 2